# FASCICULE DE BREVET EUROPEEN

(11) **EP 2 077 831 B1**
(45) Date de publication et mention de la délivrance du brevet: **24.10.2012**
(21) Numéro de dépôt: 07823875.5
(22) Date de dépôt: 27.09.2007
(51) Int. Cl.: A61K 31/357, A61P 17/00, A61P 17/10, A61P 17/08

(54) **UTILISATION DE LA 6'-ETHYL-LEPIMECTINE, LA 6'-METHYL-LÉPIMECTINE OU LEURS DERIVES POUR LE TRAITEMENT DE DESORDRES DERMATOLOGIQUES CHEZ L'HOMME**
VERWENDUNG VON 6'-ETHYL-LEPIMECTIN, 6'-METHYL-LEPIMECTIN ODER DERIVATEN DAVON ZUR BEHANDLUNG VON HAUTERKRANKUNGEN BEI MENSCHEN
USE OF 6'-ETHYL-LEPIMECTINE, 6'-METHYL-LEPIMECTINE OR DERIVATIVES THEREOF FOR THE TREATMENT OF DERMATOLOGICAL DISORDERS IN HUMAN BEINGS

(30) Priorité: 28.09.2006 FR 0653998
(43) Date de publication de la demande: 15.07.2009
(73) Titulaire: Galderma S.A., 6330 Cham (CH)
(72) Inventeur: KAOUKHOV, Alexandre, 06160 Juan Les Pins (FR); COUSIN, Cécile, 06370 Mouans-sartoux (FR)
(74) Mandataire: Boulard, Denis
(86) Numéro de dépôt international: PCT/FR2007/052040
(87) Numéro de publication internationale: WO 2008/037935

(56) Documents cités:
- WO-A-2006/069580
- US-A1- 2005 192 319
- DATABASE CA [Online] CHEMICAL ABSTRACTS SERVICE, COLUMBUS, OHIO, US; 7 septembre 2006 (2006-09-07), HOSODA, HITOSHI ET AL: "Liquid agrochemical compositions with decreased phytotoxicity and skin irritation" XP002429111 extrait de STN Database accession no. 2006:910683 & JP 2006 232709 A (SANKYO AGRO CO., LTD., JAPAN) 7 septembre 2006 (2006-09-07)

## Description

La présente invention se rapporte à l'utilisation d'au moins un composé choisi parmi la 6'-éthyl-lepimectine, la 6'-méthyl-lépimectine et ses sels pharmaceutiquement acceptables, pour la fabrication d'une composition pharmaceutique destinée au traitement d'affections dermatologiques chez l'homme, notamment la rosacée.

La rosacée est une dermatose inflammatoire commune chronique et progressive liée à une relaxation vasculaire. Elle affecte principalement la partie centrale du visage et se caractérise par le rougissement du visage ou les bouffées de chaleur, l'érythème facial, les papules, les pustules, et les télangiectasies. Dans les cas graves, particulièrement chez l'homme, un éléphantiasis facial peut se développer qui se présente le plus souvent par un enflement du tissu mou du nez produisant un gonflement bulbeux appelé rhinophyma.

La rosacée survient généralement entre l'âge de 25 et 70 ans, et elle est beaucoup plus commune chez les gens au teint clair. Elle touche plus particulièrement les femmes, bien que cette affection soit généralement plus sévère chez l'homme. La rosacée est chronique et persiste des années avec des périodes d'exacerbation et de rémission.

La pathogenèse de la rosacée est mal connue. De nombreux facteurs peuvent être impliqués sans forcément induire cette affection. Ce sont par exemple des facteurs psychologiques, des troubles gastro-intestinaux, des facteurs environnementaux (exposition au soleil, température, humidité) et émotionnels (stress), alimentaires (alcool, épices), hormonaux, vasculaires, voire une infection par *Helicobacter pilori*.

Les formes mineures de la rosacée peuvent être traitées par des traitements topiques, par exemple le metronidazole, l'acide azélaïque, le peroxyde de benzoyle, ou l'acide rétinoïque. Quant aux formes les plus sévères de l'affection, elles répondent bien à une antibiothérapie générale par les cyclines. Cependant, ces traitements présentent des effets secondaires désagréables pour le patient tels des phénomènes d'irritation ou d'intolérance.

De plus, en raison de l'aspect multi-factoriel de la rosacée, il existe de très nombreuses thérapies contre cette affection, mais on est encore à la recherche d'un traitement efficace et sans risque pour le patient.

Or, de façon surprenante, la Demanderesse a maintenant découvert que la lépimectine peut être utilisée chez l'homme, et s'avère adaptée au traitement des affections dermatologiques et plus particulièrement bien adaptée pour le traitement de la rosacée.

On appelle couramment « lépimectine » le mélange contenant au moins 80% de 6'-éthyl-lépimectine et moins de 20% de 6'-méthyl-lépimectine.

La 6'-éthyl-lepimectine et la 6'-méthyl-lépimectine sont des composés appartenant à la classe des milbémycines. La 6'-éthyl-lépimectine correspond au (10*E*,14*E*,16*E*)-(1*R*,4*S*,5'*S*,6*R*,6'*R*,8*R*,12*R*,13*S*,20*R*,21*R*,24*S*)-6'-ethyl-21,24-dihydroxy- 5',11,13,22-tetramethyl-2-oxo-(3,7,19-trioxatetracyclo[15.6.1.1^{4,8}.0^{20,24}]pentacosa-10,14,16,22-tetraene)-6-spiro-2'-(tetrahydropyran)-12-yl (*Z*)-2-methoxyimino-2-phenylacetate, et la 6'-méthyl-lépimectine correspond au (10*E*,14*E*,16*E*)-(1*R*,4*S*,5'*S*,6*R*,6'*R*,8*R*,12*R*,13*S*,20*R*,21*R*,24*S*)-21,24-di hydroxy-5',6',11,13,22-pentamethyl-2-oxo-(3,7,19-trioxatetracyclo[15.6.1.1^{4,8}.0^{20,24}]pentacosa-10.14,16,22-tetraene)-6-spiro-2'-(tetrahydropyran)-12-yl (*Z*)-2-methoxyimino-2-phenylacetate.

La lépimectine appartient au groupe des milbémycines, famille de lactones macrocycliques à activité endotectocide. Le mode d'action des milbémycines est comparable à celui des avermectines. Elles agissent sur la transmission nerveuse chez les invertébrés en potentialisant la perméabilité des membranes des nématodes et des insectes vis-à-vis des ions chlorures via les canaux glutamate-chlorure (en relation avec les récepteurs GABA_{A} et glycine). Ceci provoque une hyperpolarisation de la membrane neuromusculaire et entraîne une paralysie flasque puis la mort du parasite.

Ainsi, la présente invention a pour objet l'utilisation d'au moins un composé choisi parmi la 6'-éthyl-lepimectine, la 6'-méthyl-lépimectine et leurs sels pharmaceutiquement acceptables, pour la fabrication d'une composition pharmaceutique destinée au traitement d'affections dermatologiques chez l'homme, notamment la rosacée. De préférence, un mélange de ces composés est utilisé ; de préférence, la lépimectine est utilisée.

La présente invention s'intéresse exclusivement au traitement thérapeutique de l'homme ; en particulier, elle ne comprend pas le traitement thérapeutique d'animaux.

Les compositions pharmaceutiques ainsi obtenues sont destinées à l'usage humain et comprennent, dans un milieu pharmaceutiquement acceptable, au moins un composé choisi parmi la 6'-éthyl-lepimectine, la 6'-méthyl-lépimectine et leurs sels pharmaceutiquement acceptables, et notamment les sels formés avec un acide ou une base pharmaceutiquement acceptables.
Les acides peuvent être choisis parmi l'acide benzoïque, éventuellement substitué, l'acide benzènesulfonique, l'acide citrique, l'acide maléique, l'acide tartarique, l'acide phosphorique, l'acide salicylique et l'acide gallique.
Les bases peuvent être choisies parmi les sels de métaux alcalins et alcalino-terreux, comme les sels de lithium, calcium, sodium potassium ou magnésium, ou encore les sels d'hétérocycles aminés tels que les sels de pipéridine ou de morpholine.
Par milieu pharmaceutiquement acceptable, on entend un milieu compatible avec la peau, les muqueuses et/ou les phanères.

La composition pharmaceutique utilisable selon l'invention est destinée au traitement de la peau humaine et peut être administrée par voie topique, parentérale ou orale. De préférence, la composition est administrée par voie topique.

Par voie orale, la composition pharmaceutique peut se présenter sous forme liquide, pâteuse ou solide, sous forme de poudres et plus particulièrement sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Par voie parentérale, la composition peut se présenter sous forme de solutions ou suspensions pour perfusion ou pour injection.

Par voie topique, la composition peut se présenter sous forme liquide, pâteuse, ou solide, et plus particulièrement sous forme d'onguents, de crèmes, de laits, de pommades, de poudres, de tampons imbibés, de syndets, des lingettes, de solutions, de gels, de sprays, de mousses, de suspensions, de lotions, de sticks, de shampoings, ou de bases lavantes. Elle peut également se présenter sous forme de suspensions de microsphères ou nanosphères ou de vésicules lipidiques ou polymériques ou de patchs polymériques et d'hydrogels permettant une libération contrôlée. Cette composition pour application topique peut se présenter sous forme anhydre, sous forme aqueuse ou sous la forme d'une émulsion.

Dans une variante préférée de l'invention, la composition pharmaceutique topique selon l'invention se présente sous la forme d'une émulsion de type crème ou lotion, d'un gel, ou d'une solution.

Lorsque la composition selon l'invention est une émulsion, elle comprend au moins un agent tensio-actif. En effet, les émulsions classiques telles que décrites dans l'art antérieur sont des systèmes instables quasi-homogènes de deux liquides non miscibles dont l'un est dispersé dans l'autre sous forme de fines gouttelettes (micelles). Cette dispersion est stabilisée grâce à l'action d'agents émulsionnants tensio-actifs qui modifient la structure et le rapport des forces au niveau de l'interface, et donc augmentent la stabilité de la dispersion en diminuant l'énergie de tension interfaciale.

Les émulsionnants tensio-actifs sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.
Le pouvoir émulsionnant des tensio-actifs non-ioniques est étroitement lié à la polarité de la molécule. Cette polarité est définie par le HLB (Balance Hydrophile/Lipophile). Les émulsions classiques sont généralement stabilisées par un mélange de tensio-actifs dont les HLB peuvent être assez différents mais dont la proportion dans le mélange correspond au HLB requis de la phase grasse à émulsionner.

Parmi ces composés, on peut citer à titre d'exemples le glyceryle / PEG100 stéarate vendu sous le nom de Arlacel 165FL par la société UNIQEMA ou sous le nom de Simulsol 165 par la société SEPPIC, des esters d'acides gras polyoxyéthylénés tel que l'Arlatone 983 de la société UNIQEMA ou l'alcool stéarylique polyoxyéthyléné (2) vendu sous le nom de Brij72 associé à l'alcool stéarylique polyéthyléné (21) vendu sous le nom de Brij721 par la société UNIQEMA, les esters de sorbitan tels que l'oléate de sorbitan vendu sous le nom de Arlacel 80 par la société ICI ou vendu sous le nom de Crill 4 par la société Croda, le sesquioleate de sorbitan vendu sous le nom de Arlacel 83 par la société ICI ou vendu sous le nom de Montane 83 par la société SEPPIC, ou bien l'isostéarate de sorbitan; les éthers d'alcools gras.

La composition selon l'invention comprend avantageusement jusqu'à 15% en poids d'émulsionnant tensioactif approprié, de préférence de 2 à 12% en poids et plus particulièrement de 2 à 6% en poids par rapport au poids total de la composition.

La composition selon l'invention est avantageusement une émulsion qui comprend :
a) une phase huileuse comprenant des corps gras ;
b) au moins un émulsionnant tensio-actif ;
c) au moins un composé choisi parmi la 6'-éthyl-lepimectine, la 6'-méthyl-lépimectine et leurs dérivés ;
d) un ou plusieurs solvants et/ou propénétrants de l'actif ;
e) et de l'eau.

La phase huileuse de la composition selon l'invention peut comprendre par exemple, les huiles végétales, minérales, animales ou synthétiques, des huiles de silicones, des alcools de Guerbet ou autres corps gras et leurs mélanges.

Comme exemple d'huile minérale, on peut citer par exemple des huiles de paraffine de différentes viscosité telles que le Primol 352, le Marcol 82, Marcol 152 vendus par la société Esso.

Comme huile végétale, on peut citer l'huile d'amande douce, l'huile de palme, l'huile de soja, l'huile de sésame, l'huile de tournesol.

Comme huile animale, on peut citer la lanoline, le squalene, l'huile de poisson, l'huile de vison.

Comme huile synthétique, on peut citer des esters, tel que le cetearyl isononanoate vendu sous le nom notamment de Cetiol SN par la société Cognis France, le diisopropyl adipate comme le produit vendu sous le nom de Ceraphyl 230 par la société ISF, le palmitate d'isopropyle comme le produit vendu sous le nom de Crodamol IPP par la société Croda, le caprylique caprique triglyceride tel que Miglyol 812 vendu par la société Huls / Lambert Rivière.

Comme huile de silicone, on peut citer une dimethicone comme le produit vendu sous le nom de Dow Corning 200 fluid, une cyclomethicone comme le produit vendu sous le nom de Dow Corning 244 fluid par la société Dow Corning ou le produit vendu sous le nom le Mirasil CM5 par la société SACI-CFPA.

Comme autres corps gras on peut citer des acides gras tel que l'acide stéarique, les alcools gras tels que l'alcool stearylique, l'alcool cetostéarylique et l'alcool cétylique ou leurs dérivés, les cires telles que cire d'abeille, cire de carnauba, cire de candellilla, ainsi que des gommes, en particuliers des gommes de silicone.

Les ingrédients de la phase huileuse pourront être choisis de manière variée par l'homme du métier afin de préparer une composition ayant les propriétés souhaitées, par exemple en consistance ou en texture.

De préférence, la phase huileuse de la composition selon l'invention comprend une huile synthétique et/ou une huile de silicone, comme huile synthétique, on préfère le palmitate d'isopropyle comme le produit vendu sous le nom de Crodamol IPP par la société Croda ou le myristate d'isopropyl comme le produit vendu sous le nom de Crodamol IPM par la société Croda, comme huile de silicone, on préfère une diméthicone.

La phase huileuse de l'émulsion selon l'invention peut être présente à une teneur comprise entre 3 et 50 % en poids par rapport au poids total de la composition et de préférence comprise entre 6 et 20 % en poids.

La composition selon l'invention comprend de 0,001 à 10 % d'au moins un composé choisi parmi la 6'-éthyl-lepimectine, la 6'-méthyl-lépimectine et leurs sels pharmaceutiquement acceptables, de préférence de lépimectine, en poids par rapport au poids total de la composition. De manière préférentielle, la composition selon l'invention comprend de 0.1 à 5 % d'au moins un composé choisi parmi la 6'-éthyl-lepimectine, la 6'-méthyl-lépimectine et leurs sels pharmaceutiquement acceptables, de préférence de lépimectine, en poids par rapport au poids total de la composition.

A titre d'exemple de solvant et/ou propénétrant de la 6'-éthyl-lepimectine, de la 6'-méthyl-lépimectine ou leurs sels pharmaceutiquement acceptables, on citera préférentiellement le propylène glycol, les alcools du type éthanol, isopropanol, butanol, la N-methyl 2 pyrrolidone ou le DMSO, le polysorbate 80, le phénoxyéthanol et leurs mélanges.

La composition de l'invention contient de 0.1 % à 20% et préférentiellement de 1% à 10% d'un solvant et/ou propénétrant de la 6'-éthyl-lepimectine, de la 6'-méthyl-lépimectine ou leurs sels pharmaceutiquement acceptables.

La composition de l'invention contient également de l'eau allant de 30 à 95% et préférentiellement de 60 à 80% en poids par rapport au poids total de la composition. L'eau utilisée dans la composition selon l'invention sera de préférence de l'eau purifiée.

La composition selon l'invention peut également se présenter sous forme de gel ; elle comprend alors un ou plusieurs composés gélifiants, allant de 0,01 à 5% en poids par rapport au poids total de la composition. Parmi les gélifiants utilisables dans la composition selon l'invention, on peut citer les polymères carboxyvinyliques (carbomers) et, à titre d'exemples non limitatifs de carbomer, le Carbopol 981, le Carbopol ETD 2020, le Carbopol 980, le Carbopol Ultrez 10 NF, le Pemulen TR1 vendus par la société NOVEON.
On peut également citer les dérivés cellulosiques, comme par exemple l'hydroxypropylmethylcellulose, ou l'hydroxyethylcellulose; les gommes de xanthane, les silicates d'aluminium / magnesium comme le Veegum K ou le Veegum Ultra revendues par Vanderbilt, les gommes guar et semblables, les polyacrylamides tel que le mélange polyacrylamide / isoparaffine C13-14 / laureth-7 comme par exemple celui vendu par la société SEPPIC sous le nom de Sepigel 305 ou le mélange acrylamide, AMPS copolymer dispersion 40% / isohexadecane sous le nom de Simulgel 600PHA, ou la famille des amidons modifiés tel que Structure Solanace revendu par National Starch ou leurs mélanges.

La composition de l'invention contient préférentiellement de 0.01% à 5%, et, de préférence, de 0.1 à 3% de gélifiant.

Lorsque la composition se présente sous forme de solution, elle comprend, outre la 6'-éthyl-lepimectine, la 6'-méthyl-lépimectine ou leurs sels pharmaceutiquement acceptables, une solution aqueuse ou huileuse, et éventuellement un ou plusieurs solvants et/ou propénétrants de l'actif tels que décrits ci-dessus.

La composition pharmaceutique selon l'invention pourra en outre contenir des additifs inertes ou des combinaisons de ces additifs, tels que
- des agents conservateurs;
- des agents stabilisants ;
- des agents régulateurs d'humidité ;
- des agents régulateurs de pH ;
- des agents modificateurs de pression osmotique ;
- des filtres UV-A et UV-B ;
- et des antioxydants.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

Ces additifs peuvent être présents dans la composition de 0.001 à 20% en poids par rapport au poids total de la composition.

L'invention a également pour objet l'utilisation de la composition selon l'invention pour la fabrication d'une préparation pharmaceutique destinée à traiter les affections dermatologiques.

L'utilisation de la 6'-éthyl-lepimectine, la 6'-méthyl-lépimectine ou leurs sels pharmaceutiquement acceptables, de préférence la lépimectine, pour la fabrication d'une composition pharmaceutique topique à usage humain selon l'invention est destinée au traitement de la rosacée, de l'acné vulgaire, de la dermite séborrhéique, de la dermatite periorale, des éruptions acnéiformes, de la dermatite acantholytique transitoire, et de l'acné miliaris necrotica.

L'utilisation de la 6'-éthyl-lepimectine, la 6'-méthyl-lépimectine ou leurs sels pharmaceutiquement acceptables, de préférence la lépimectine, pour la fabrication d'une composition pharmaceutique topique à usage humain selon l'invention est plus particulièrement destinée au traitement de la rosacée.

Il va être maintenant donné, à titre d'illustration , diverses formulations de compositions comprenant de la 6'-éthyl-lepimectine, la 6'-méthyl-lépimectine ou leurs sels pharmaceutiquement acceptables.

### EXEMPLE 1 : Composition 1

| Ingrédients | % en poids par rapport au poids total de la composition |
|---|---|
| Lépimectine | 1.00 |
| EDTA | 0.1 |
| Polysorbate 80 | 8.0 |
| Propylene Glycol | 20.00 |
| Alcool benzylique | 3 |
| Eau | Qsp 100 |

### EXEMPLE 2 : Composition 2

| Ingrédients | % en poids par rapport au poids total de la composition |
|---|---|
| Lépimectine | 1.00 |
| Vaseline Codex | 56.00 |
| Huile de vaseline | 43.00 |

### EXEMPLE 3 : Composition 3

| Ingrédients | % en poids par rapport au poids total de la composition |
|---|---|
| Lépimectine | 1.00 |
| Glycérine | 4.0 |
| Steareth-2 | 1.0 |
| Steareth-21 | 2.0 |
| Silicate d'aluminium et de magnésium/ dioxyde de titane/silice | 1.0 |
| Parahydroxybenzoate de méthyle | 0.2 |
| Parahydroxybenzoate de propyle | 0.1 |
| EDTA disodique | 0.05 |
| Acide Citrique monohydrate | 0.05 |
| Palmitate d'Isopropyle | 4.0 |
| Glyceryle/PEG 100 stéarate | 2.0 |
| Cire autoémulsionnable | 1.0 |
| Acide palmitostéarique | 2.00 |
| Dimethicone 200-350 cS | 0.5 |
| Propylene Glycol | 4.0 |
| Triacetate de glycérol | 1.00 |
| Phenoxyethanol | 0.5 |
| Hydroxyde de Sodium 10% | Qs pH |
| Eau | Qsp 100 |

## Revendications

1. Utilisation d'au moins un composé choisi parmi la 6'-éthyl-lepimectine, la 6'-méthyl-lépimectine et leurs sels formés avec un acide ou une base pharmaceutiquement acceptables pour la fabrication d'une composition pharmaceutique destinée au traitement des affections dermatologiques chez l'homme choisies parmi la rosacée, l'acné vulgaire, la dermite séborrhéique, la dermatite periorale, les éruptions acnéiformes, la dermatite acantholytique transitoire et l'acné miliaris necrotica.

2. Utilisation selon la revendication 1, **caractérisée en ce que** la composition pharmaceutique comprend un mélange d'au moins 80% de 6'-éthyl-lépimectine ou ses sels formés avec un acide ou une base pharmaceutiquement acceptables avec moins de 20% de 6'-méthyl-lépimectine ou sels formés avec un acide ou une base pharmaceutiquement acceptables.

3. Utilisation selon la revendication 1 ou 2, **caractérisée en ce que** la composition pharmaceutique comprend la lépimectine.

4. Utilisation selon l'une des revendications 1 à 3, **caractérisée en ce que** la composition se présente sous une forme adaptée pour une application orale.

5. Utilisation selon l'une des revendications 1 à 3, **caractérisée en ce que** la composition se présente sous une forme adaptée pour une application topique.

6. Utilisation selon la revendication 5, **caractérisée en ce que** la composition se présente sous la forme d'une émulsion, d'un gel ou d'une solution.

7. Utilisation selon l'une quelconque des revendications 1 à 6, **caractérisée en ce que** la composition comprend de 0.001% à 10% en poids de 6'-éthyl-lepimectine, 6'-méthyl-lépimectine ou leurs sels formés avec un acide ou une base pharmaceutiquement acceptables par rapport au poids total de la composition.

8. Utilisation selon l'une des revendications 1 à 9, **caractérisée en ce que** l'affection dermatologique est la rosacée.

## Claims

1. Use of at least one compound chosen from 6'-ethyl lepimectin, 6'-methyl lepimectin and salts thereof formed with a pharmaceutically acceptable acid or base for the manufacture of a pharmaceutical composition for use in the treatment of dermatological conditions in humans, chosen from rosacea, common acne, seborrhoeic dermatitis, perioral dermatitis, acneiform rashes, transient acantholytic dermatitis and acne necrotica miliaris.

2. Use according to Claim 1, **characterized in that** the pharmaceutical composition comprises a mixture of at least 800 of 6'-ethyl lepimectin or salts thereof formed with a pharmaceutically acceptable acid or base with less than 20% of 6'-methyl lepimectin or salts formed with a pharmaceutically acceptable acid or base.

3. Use according to Claim 1 or 2, **characterized in that** the pharmaceutical composition comprises lepimectin.

4. Use according to one of Claims 1 to 3, **characterized in that** the composition is in a form suitable for oral application.

5. Use according to one of Claims 1 to 3, **characterized in that** the composition is in a form suitable for topical application.

6. Use according to Claim 5, **characterized in that** the composition is in the form of an emulsion, a gel or a solution.

7. Use according to any one of Claims 1 to 6, **characterized in that** the composition comprises from 0.001% to 10% by weight of 6'-ethyl lepimectin, 6'-methyl lepimectin or salts thereof formed with a pharmaceutically acceptable acid or base, relative to the total weight of the composition.

8. Use according to one of Claims 1 to 7, **characterized in that** the dermatological condition is rosacea.

## Patentansprüche

1. Verwendung von mindestens einer Verbindung aus der Reihe 6'-Ethyllepimectin, 6'-Methyllepimectin und ihren mit einer pharmazeutisch unbedenklichen Säure oder Base gebildeten Salzen für die Herstellung einer pharmazeutischen Zusammensetzung zur Behandlung von Hauterkrankungen beim Menschen aus der Reihe Rosazea, Acne vulgaris, Dermatitis seborrhoica, periorale Dermatitis, akneiforme Eruptionen, transitorische akantholytische Dermatose sowie Acne miliaris necrotica.

2. Verwendung nach Anspruch 1, **dadurch gekennzeichnet, dass** die pharmazeutische Zusammensetzung eine Mischung von mindestens 80% 6'-Ethyllepimectin oder seinen mit einer pharmazeutisch unbedenklichen Säure oder Base gebildeten Salzen und mindestens 20% 6'-Methyllepimectin oder mit einer pharmazeutisch unbedenklichen Säure oder Base gebildeten Salzen umfasst.

3. Verwendung nach Anspruch 1 oder 2, **dadurch gekennzeichnet, dass** die pharmazeutische Zusammensetzung Lepimectin umfasst.

4. Verwendung nach einem der Ansprüche 1 bis 3, **dadurch gekennzeichnet, dass** die Zusammensetzung in einer an den oralen Verabreichungsweg angepassten Form vorliegt.

5. Verwendung nach einem der Ansprüche 1 bis 3, **dadurch gekennzeichnet, dass** die Zusammensetzung in einer an den topischen Verabreichungsweg angepassten Form vorliegt.

6. Verwendung nach Anspruch 5, **dadurch gekennzeichnet, dass** die Zusammensetzung in Form einer Emulsion, eines Gels oder einer Lösung vorliegt.

7. Verwendung nach einem der Ansprüche 1 bis 6, **dadurch gekennzeichnet, dass** die Zusammensetzung 0,001 Gew.-% bis 10 Gew.-% 6'-Ethyllepimectin, 6'-Methyllepimectin oder ihre mit einer pharmazeutisch unbedenklichen Säure oder Base gebildeten Salze in Bezug auf das Gesamtgewicht der Zusammensetzung umfasst.

8. Verwendung nach einem der Ansprüche 1 bis 7, **dadurch gekennzeichnet, dass** es sich bei der Hauterkrankung um Rosazea handelt.
